## Europäisches Patentamt
## European Patent Office
## Office européen des brevets

(19)

(11) Veröffentlichungsnummer: **0 144 760**
**B1**

(12)

# EUROPÄISCHE PATENTSCHRIFT

(45) Veröffentlichungstag der Patentschrift:
04.01.89

(21) Anmeldenummer: 84113307.7

(22) Anmeldetag: 06.11.84

(51) Int. Cl.⁴: **C 07 C 37/62**, C 07 C 39/28, C 07 C 39/30

(54) **Verfahren zur Isomerisierung von Mono- oder Dichlorophenolen.**

(30) Priorität: 12.11.83 DE 3340997
17.12.83 DE 3345808

(43) Veröffentlichungstag der Anmeldung:
19.06.85 Patentblatt 85/25

(45) Bekanntmachung des Hinweises auf die Patenterteilung:
04.01.89 Patentblatt 89/1

(84) Benannte Vertragsstaaten:
BE CH DE FR GB IT LI NL

(56) Entgegenhaltungen:
EP-A-0 012 570
GB-A-2 012 271
US-A-3 655 780

PATENTS ABSTRACTS OF JAPAN, Band 4, Nr. 93 (C-17) 575 , 5. Juli 1980; & JP-A-55 55 128 (SANKOU KAGAKU K.K.) 22-04-1980

(73) Patentinhaber: HOECHST AKTIENGESELLSCHAFT,
Postfach 80 03 20, D-6230 Frankfurt am Main 80
(DE)

(72) Erfinder: Baltes, Herbert, Dr., Johannesallee 24,
D-6230 Frankfurt am Main 80 (DE)
Erfinder: Leupold, Ernst Ingo, Dr., Am Zäunefeld
15, D-6392 Neu- Anspach (DE)

EP 0 144 760 B1

## Beschreibung

Die Erfindung betrifft ein Verfahren zur Isomerisierung von Mono- oder Dichlorphenolen.

Monochlorphenole stellt man technisch im allgemeinen durch Chlorierung von Phenol dar. Dabei entstehen Gemische aus 2- und 4-Chlorphenol. Das Verhältnis dieser beiden Isomeren läßt sich dabei in weiten Grenzen durch die Wahl geeigneter Reaktionsbedingungen beeinflussen (Houben-Weyl, Methoden der Organischen Chemie, Band V/3, Halogenverbindungen, Stuttgart 1962, S. 679).

3-Chlorphenol ist auf diese Weise nicht zugänglich, sondern wird durch Diazotieren und Verkochen aus 3-Chloranilin dargestellt (Ullmanns Enzyklopädie der Technischen Chemie, 4. Auflage, Band 9, Seite 574). Diese mehrstufige Synthese ist umständlich und kostspielig.

Einfache und ökonomische Methoden im großtechnischen Maßstab zur Herstellung von 3-Chlorphenol werden bisher nicht beschrieben.

Auch Dichlorphenole stellt man technisch im allgemeinen durch Kernchlorierung von Phenol dar. Dabei entsteht zunächst ein Gemisch aus o- und p-Chlorphenol, dessen Weiterchlorierung zu 2,4-Dichlorphenol und - in geringerem Maße - zu 2,6-Dichlorphenol führt (Houben-Weyl, Methoden der Organischen Chemie, Band V/3, Halogenverbindungen, Stuttgart 1962, S. 680).

Die anderen vier Isomeren des Dichlorphenols sind durch direkte Chlorierung nicht zugänglich. Ihre Herstellung erfordert zum Teil aufwendige und kostspielige mehrstufige Synthenen. 2,3-Dichlorphenol beispielsweise ist aus 1,2,3-Trichlorbenzol durch Sulfonierung und anschließende Hydrolyse zugänglich. 3,4-Dichlorphenol läßt sich oxidativ aus 3,4-Dichlorcumol synthetisieren; weitere Methodon sind die Diazotierung nach Sandmeier, die partielle Hydrolyse von Trichlorbenzolen und die partielle Hydrierung von polychlorierten Phenolen (Kirk-Othmer, Encyclopedia of Chemical Technology, 3. Auflage, Band 5, S. 866).

Einfache und ökonomische Methoden im großtechnischen Maßstab zur Herstellung von 2,3-Dichlorphenol, 2,5-Dichlorphenol, 3,4-Dichlorphenol und 3,5-Dichlorphenol wurden bisher nicht beschrieben.

Gegenstand der vorliegenden Erfindung ist ein Verfahren zur Isomerisierung von Mono- oder Dichlorphenolen, dadurch gekennzeichnet, daß man mindestens ein Mono- oder Dichlorphenol in der Gasphase über einen Zeolithkatalysator leitet. Insbesonders ist Gegenstand der vorliegenden Erfindung ein Verfahren zur Herstellung von 3-Chlorphenol, dadurch gekennzeichnet, daß man 2-Chlorphenol und/oder 4-Chlorphenol in der Gasphase über einen Zeolithkatalysator leitet. Ein weiterer Gegenstand der vorliegenden Erfindung ist ein Verfahren zur Herstellung von 2,5-Dichlorphenol durch Isomerisierung von 2,4-Dichlorphenol in der Gasphase an einem Zeolithkatalysator.

Aufgrund des Standes der Technik war es überraschend und in keiner Weise vorhersehbar, daß sich Monochlorphenole in so einfacher Weise isomerisieren lassen, und vor allem daß sich das bisher schwer herstellbare 3-Chlorphenol so leicht aus technisch gut zugänglichem 2-Chlorphenol, 4-Chlorphenol oder deren Gemischen herstellen läßt.

Zur Isomerisierung der Monochlorphenole wird eines der Monochlorphenole oder ein Gemisch aus zweien oder ein Gemisch aus allen drei Monochlorphenolen entweder alleine oder zusammen mit einem oder mehreren organischen Verdünnungsmitteln gasförmig über den Zeolithkatalysator geleitet. Besonders wichtig ist der Einsatz des Isomerengemisches, das bei der Chlorierung von Phenol entsteht.

Ebenso war es überraschend, daß sich Dichlorphenole in so einfacher Weise isomerisieren lassen, und daß sich bisher schwer herstellbare Dichlorphenole so leicht aus technisch gut zugänglichen Dichlorphenolen herstellen lassen, wie 2,5-Dichlorphenol aus 2,4-Dichlorphenol.

Zur Isomerisierung der Dichlorphenole wird eines der Dichlorphenole oder ein Gemisch von Dichlorphenolen entweder alleine oder zusammen mit einem oder mehreren organischen Verdünnungsmitteln gasförmig über den Zeolithkatalysator geleitet. Das wichtigste Ausgangsmaterial ist das im großtechnischen Maßstab verfügbare 2,4-Dichlorphenol oder ein Gemisch der Dichlorphenole, wie es bei der Phenolchlorierung entsteht.

Als organische Verdünnungsmittel eignen sich insbesondere aromatische Kohlenwasserstoffe, vorzugsweise Chlorbenzol, Benzol und/oder Toluol. Das Molverhältnis des Verdünnungsmittels zu Dichlorphenol beträgt im allgemeinen 0 : 1 bis 10 : 1, vorzugsweise 0 : 1 bis 3 : 1.

Als Zeolithe eignen sich im allgemeinen sowohl natürliche wie auch synthetische Zeolithe, vorzugsweise synthetische Zeolithe vom Pentasil-, Mordenit- oder Faujasit-Typ, insbesondere synthetische Zeolithe vom Pentasil-Typ.

Für den Begriff Pentasile gilt dabei die Definition von Kokotailo und Meier ("Pentasil family of high silicon crystalline materials" in Special Publication No. 33 of the Chemical Society, London, 1980). Die Pentasil-Familie umfaßt beispielsweise die synthetischen Zeolithe ZSM-5 (US-PS-3 702 886), ZSM-8 (GB-PS-1 334 243), ZSM-11 (US-PS-3 709 979) und ZSM-23 (US-PS-4 076 842).

Das Si/Al-Verhältnis der Pentasile liegt vorzugsweise bei 20 bis 2000, das der Mordenite bei vorzugsweise 5 bis 100. Pentasile oder Mordenite mit einem höheren Aluminiumgehalt lassen sich dabei auf das gewünschte Si/Al-Verhältnis einstellen, indem man durch Behandlung mit Mineralsäuren, organischen Säuren oder chelatisierenden Substanzen einen Teil des Aluminiums aus dem Zeolith-Gitter entfernt.

Bei dem erfindungsgemäßen Verfahren werden die Zeolithe im allgemeinen in ihrer sauren Form eingesetzt. Diese sauren Formen lassen sich nach bekannten Methoden aus den Alkalimetall-Formen, wie sie in der Regel bei der Zeolith-Synthese anfallen bzw. als Naturprodukte vorkommen, durch vollständigen oder partiellen Ionenaustausch herstellen. Die bevorzugten sauren Formen sind teilweise oder vollständig ausgetauschte H- oder Ammoniumformen, insbesondere H-Formen. Eine übliche Methode zur Herstellung der H-Form eines

2

Zeoliths besteht beispielsweise darin, die Alkalimetall-Form zunächst durch partiellen oder vollständigen Ionenaustausch mit einer Ammoniumsalz-Lösung in die Ammoniumform und diese anschließend durch Kalzinieren in die H-Form zu überführen. Aber auch die mit Alkali-, Erdalkali- und mit Seltenerdmetall-Ionen teilweise oder vollständig ausgetauschten Formen zeigen katalytische Aktivität.

Die erfindungsgemäßen Zeolithkatalysatoren bestehen im allgemeinen aus der katalytisch aktiven Zeolith-Komponente sowie einem Bindematerial. Letzteres ist erforderlich, um den Zeolith in eine für das erfindungsgemäße Verfahren geeignete äußere Form zu bringen.

Als Bindematerialien eignen sich vor allem Oxide oder Hydroxide des Aluminiums, des Siliciums und des Titans, sowie Schichtsilicate, beispielweise aus der Kaolin- oder Montmorillonit-Familie.

Dieser so hergestellte Zeolithkatalysator wird gewöhnlich vor dem Einsatz in der erfindungsgemäßen Isolerisierungsreaktion zunächst durch Kalzinieren bei Temperaturon zwischen 300 und 700°C aktiviert. Zur besseren Stabilisierung des Katalysators ist es manchmal vorteilhaft, die Kalzinierung in Gegenwart von Wasserdampf, Ammoniak oder deren Mischungen durchzuführen.

Es hat sich als vorteilhaft erwiesen, dem gasförmigen Ausgangsmaterial (mindestens ein Mono- oder Dichlorphenol) bzw. dem gasförmigen Gemisch aus Ausgangsmaterial und dem organischen Verdünnungsmittel zusätzlich noch Wasserstoff zuzumischen. Darüberhinaus kann es noch vorteilhaft sein, ein unter den Reaktionsbedingungen inertes Trägergas zuzumischen. Als Trägergase eignen sich z. B. Stickstoff und Edelgase. Wasserstoff und/oder das Trägergas wird dabei in einer Menge zugesetzt, daß die Verweilzeit zwischen 1 und 10 s liegt.

Die erfindungsgemäßen Isomerisierung wird im allgemeinen bei Temperaturen zwischen 300 und 550°C, vorzugsweise bei 320 bis 450°C, sowie bei Drücken von 0,1 bis 100 bar, vorzugsweise bei 1 - 40 bar, insbesondere bei Normaldruck durchgeführt.

Die Belastung des Zeolithkatalysators - ausgedrückt als LHSV (Liquid Hourly Space Velocity, $h^{-1}$) - liegt dabei im allgemeinen zwischen 0,1 und 10 $h^{-1}$, vorzugweise zwischen 0,3 und 5 $h^{-1}$.

Eine günstige einfache Verfahrensweise zur Durchführung der erfindungsgemäßen Isomerisierung der Monochlorphenole besteht darin, daß das Ausgangsmaterial bzw. das Gemisch aus Ausgangsmaterial und dem organischen Verdünnungsmittel aus einer Dosiervorrichtung in eine Verdampfungszone und das entstandene Gas dann durch ein von außen beheiztes und mit dem Katalysator gefülltes Reaktionsrohr geleitet wird.

Eine günstige einfache Verfahrensweise zur Durchführung der erfindungsgemäßen Isomerisierung der Dichlorphenole besteht darin, daß das Ausgangsmaterial als Schmelze, bzw. als Lösung in einem organischen Verdünnungsmittel aus einer Dosiervorrichtung in eine Verdampfungszone und das entstandene Gas dann durch ein von außen beheiztes und mit dem Katalysator gefülltes Reaktionsrohr geleitet wird.

In der Verdampfungszone erfolgt gegebenenfalls die Vermischung mit dem Wasserstoff und/oder dem inerten Trägergas; es hat sich dabei als vorteilhaft erwiesen, diese Gase vor der Vermischung auf die Reaktionstemperatur aufzuheizen. Die Reaktionsprodukte werden nach Verlassen des Reaktors zur Abtrennung der kondensierbaren Anteile gekühlt. Die erfindungsgemäße Isomerisierung ist jedoch nicht auf diese Verfahrensweise (Festbett-Reaktor) beschränkt, sondern läst sich im Prinzip auch in anderen, für Gasphasen-Reaktionen geeigneten Reaktor-Typen durchführen (z. B. Wirbelschicht-Reaktor).

Die entstehenden Isomerengemische können nach bekannten Verfahren getrennt werden (durch Destillation, fraktionierte Kristallisation, Extraktion bzw. durch Kombination dieser Methoden).

Die Erfindung soll durch die folgenden Beispiele erläutert werden, wobei die Belspiele aber in keiner Weise einschränkend sein sollen.

## Beispiel 1

### A. Herstellung des Katalysators

Pulverförmiger Zeolith ZSM-5 mit einem Si/Al-Verhältnis von 24 : 1 wurde unter Zusatz von 40 Gew.-% $Al_2O_3$ als Bindemittel in Form von Strangpreßlingen extrudiert, bei 120°C für 12 h getrocknet und bei 500°C 4 h im Luftstrom kalziniert. Anschließend wurden die Strangpreßlinge 5-mal mit 10 gew.-%-iger Ammoniumchlorid-Lösung bei 90°C ausgetauscht, gründlich mit Wasser gewaschen, 12 h bei 120°C getrocknet und nochmals 4 h in Luft bei 500°C kalziniert. Die Strangpreßlinge wurden danach zerkleinert. Für die Isomerisierungsversuche wurde eine Kornfraktion von 1,0 bis 1,5 mm Durchmesser ausgesiebt. Vor dem jeweiligen Isomerisierungsversuch wurde der Katalysator noch 2 h bei 450°C aktiviert.

### B. Durchführung der Isomerisierung

9 ml/h 2-Chlorphenol wurden mit Hilfe einer Kolbenspritze über eine Verdampfungszone in einen senkrecht angeordneten Glasreaktor von 150 mm Länge und 20 mm Durchmesser eingeleitet. Gleichzeitig führte man der Verdampfungszone 3 l/h Wasserstoff zu, welcher vorher auf 400°C aufgeheizt wurde.

Der Raktor wurde von außen ebenfalls auf 400°C geheizt und war mit 15 ml des oben beschriebenen Zeolithkatalysators (H-ZSM-5) gefüllt. Die Temperatur im Innern des Reaktors wurde mit Hilfe eines Thermoelementes gemessen. Die Reaktionsprodukte wurden in einer Kühlfalle bei -70°C kondensiert.

Nach einer Anlaufzeit von 1 h zur Einstellung konstanter Betriebsbedingungen wurde der eigentliche Katalysatortest über einen Zeitraum von 2 h durchgeführt. Das Kohdensat wurde gaschromatographisch analysiert.

Die Zusammensetzung des produktionsgemisches sowie die Selektivitäten gehen aus der Tabelle 1 hervor.

**Beispiel 2**

12 ml/h einer Lösung aus 50 Gew.-% 4-Chlorphenol und 50 Gew.-% Chlorbenzol wurden zusammen mit 5 l/h Stickstoff analog zu Beispiel 1 in die dort beschriebene Apparatur eingeleitet. Der Reaktor enthielt 15 ml H-ZSM-5 wie in Beispiel 1 und wurde auf 380°C geheizt. Das Ergebnis eines zweistündigen Versuches geht aus Tabelle 1 hervor.

**Beispiel 3**

Analog Beispiel 1 wurde 15 ml/h 2-Chlorphenol und 10 l/h Wasserstoff bei 400°C über 15 ml H-Mordenit geleitet. Das Ergebnis eines zweistündigen Versuches geht aus Tabelle 1 hervor.

**Tabelle 1**

| | Beispiel 1 2-Chlorphenol | Beispiel 2*) 4-Chlorphenol | Beispiel 3 2-Chlorphenol |
|---|---|---|---|
| Ausgangsisomeres: Zusammensetzung des Endprodukts (Gew.-%) | | | |
| 2-Chlorphenol | 48,2 | 3,1 | 67,4 |
| 3-Chlorphenol | 34,4 | 17,3 | 23,4 |
| 4-Chlorphenol | 7,4 | 28,4 | 3,4 |
| Phenol | 6,4 | 2,1 | 1,1 |
| Selektivität für 3-Chlorphenol (%) | 65,3 | 68,3 | 70,6 |

*) Im Produktgemisch sind noch 47,2 Gew.-% Chlorbenzol (Verdünnungsmittel) enthalten.

**Beispiel 4**

**A. Herstellung des Katalysators**

Wie in Beispiel 1

**B. Durchführung der Isomerisierung**

9 ml/h einer Lösung aus 50 Gew.-% 2,4-Dichlorphenol und 50 Gew.-% Chlorbenzol wurden mit Hilfe einer Kolbenspritze über eine Verdampfungszone in einen senkrecht angeordneten Glasreaktor von 150 mm Länge und 20 mm Durchmesser eingeleitet. Gleichzeitig führte nan der Verdampfungszone 3 l/h Wasserstoff zu, welcher vorher auf 400°C aufgeheizt wurde.

Der Reaktor wurde von außen ebenfalls auf 400°C geheizt und war mit 15 ml des oben beschriebenen Zeolithkatalysators (H-ZSM-5) gefüllt. Die Temperatur im Innern des Reaktors wurde mit Hilfe eines Thermoelementes gemessen. Die Reaktionsprodukte wurden in einer Kühlfalle bei -70°C kondensiert.

Nach einer Anlaufzeit von 1 h zur Einstellung konstanter Betriebsbedingungen wurde der eigentliche Katalysatortest über einen Zeitraum von 2 h durchgeführt. Das Kondensat wurde flüssigkeitschromntographisch analysiert.

Das Ergebnis geht aus Tabelle 2 hervor.

**Beispiel 5**

Ca. 6 ml/h 2,4-Dichlorphenol wurden als Schmelze aus einem auf 70°C geheizten Tropftrichter in einen Verdampfer geleitet und bei 350°C verdampft. Im Verdampfer erfolgte eine Vermischung des 2,4-Dichlorphenoldampfes mit Stickstoff (10 l/h). Dieses Gemish wurde dann in den in Beispiel 4 beschriebenen und mit H-ZSM-5 gefüllten Reaktor geleitet. Der Reaktor wurde auf 380°C gcheizt. Das Ergebnis eines zweistündigen Versuches geht aus Tabelle 2 hervor.

**Beispiel 6**

12 ml/h einer 50 gew.-%-igen Lösung aus 2,5-Dichlorphenol in Chlorbenzol wurden zusammen nit 5 l/h Stickstoff analog zu Beispiel 4 in die dort beschriebene Apparatur eingeleitet. Der Reaktor enthielt 15 ml H-ZSM-5 wie in Beispiel 4 und wurde auf 400°C geheizt. Das Ergebnis eines zweistündigen Versuches geht aus Tabelle 2 hervor.

**Beispiel 7**

9 ml/h einer 40 Gew.-%-igen Lösung aus 2,3-Dichlorphenol in Chlorbenzol wurden zusammen mit 5 l/h Stickstoff analog zu Beispiel 4 in die dort beschriebene Apparatur eingeleitet. Der Reaktor enthielt 15 ml H-ZSM-5 und wurde auf 430°C geheizt. Das Ergebnis eines zweistündigen Versuches geht aus Tabelle 2 hervor.

**Tabelle 2**

| Ausgangsisomeres: | Beispiel 4 2,4-Dichlorphenol | Beispiel 5 2,4-Dichlorphenol | Beispiel 6 2,5-Dichlorphenol | Beispiel 7 2,3-Dichlorphenol |
|---|---|---|---|---|
| Ausbeute (Gew.-%) der gebildeten Isomeren: | | | | |
| 2,3-Dichlorphenol | 0,6 | 0,5 | 3,5 | A[1] |
| 2,4-Dichlorphenol | A[1] | A[1] | 15,4 | 4,0 |
| 2,5-Dichlorphenol | 26,4 | 8,3 | A[1] | 8,0 |
| 2,6-Dichlorphenol | < 0,1 | < 0,1 | < 0,1 | < 0,1 |
| 3,4-Dichlorphenol | < 0,1 | < 0,1 | 2,8 | < 0,1 |
| 3,5-Dichlorphenol | < 0,1 | < 0,1 | < 0,1 | < 0,1 |
| Chlorphenole | 4,2 | 1,2 | 5,2 | 2,2 |
| Selektivität (%) für Dichlorphenole | 67,4 | 64,2 | 68,1 | 66,3 |

A[1] = Ausgangsisomeres

**Patentansprüche**

1. Verfahren zur Isomerisierung von Mono- oder Dichlorphenolen, dadurch gekennzeichnet, daß man mindestens ein Mono- oder Dichlorphenol in der Gasphase über einen Zeolithkatalysator leitet.

2. Verfahren zur Isomerisierung von Monochlorphenolen, dadurch gekennzeichnet, daß man mindestens ein Chlorphenol in der Gasphase über einen Zeolithkatalysator leitet.

3. Verfahren nach Anspruch 2, dadurch gekennzeichnet, daß man Gemische aus 2-Chlorphenol und 4-Chlorphenol einsetzt.

4. Verfahren zur Herstellung von 3-Chlorphenol, dadurch gekennzeichnet, daß man 2-Chlorphenol und/oder 4-Chlorphenol in der Gasphase über einen Zeolithkatalysator leitet.

5. Verfahren zur Isomerisierung von Dichlorphenolen, dadurch gekennzeichnet, daß man mindestens ein Dichlorphenol in der Gasphase über einen Zeolithkatalysator leitet.

6. Verfahren zur Herstellung von 2,5-Dichlorphenol, dadurch gekennzeichnet, daß man 2,4-Dichlorphenol in der Gasphase über einen Zeolithkatalysator leitet.

7. Verfahren nach einer der Ansprüche 1 bis 6, dadurch gekennzeichnet, daß man einen Zeolith vom Pentasil-, Mordenit- oder Faujasit-Typ einsetzt.

8. Verfahren nach einem der Ansprüche 1 bis 6, dadurch gekennzeichnet, daß man einen sauren Zeolith vom Pentasil-, Mordenit- oder Faujasit-Typ einsetzt.

9. Verfahren nach einem der Ansprüche 1 bis 6, dadurch gekennzeichnet, daß man einen sauren Zeolith vom Pentasil-Typ einsetzt.

10. Verfahren nach einem der Ansprüche 8 und 9, dadurch gekennzeichnet, daß der saure Zeolith Protonen als Kationen enthält.

11. Verfahren nach einem der Ansprüche 1 bis 10, dadurch gekennzeichnet, daß man bei Temperaturen zwischen 300 und 550°C arbeitet.


**Claims**

1. A process for isomerizing monochlorophenols or dichlorophenols, which comprises passing at least one monochlorophenol or dichlorophenol in the gas phase over a zeolite catalyst.

2. A process for isomerizing monochlorophenols, which comprises passing at least one chlorophenol in the gas phase over a zeolite catalyst.

3. The process as claimed in claim 2, wherein mixtures of 2-chlorophenol and 4-chlorophenol are used.

4. A process for preparing 3-chlorophenol, which comprises passing 2-chlorophenol and/or 4-chlorophenol in the gas phase over a zeolite catalyst.

5. A process for isomerizing dichlorophenols, which comprises passing at least one dichlorophenol in the gas phase over a zeolite catalyst.

6. A process for preparing 2,5-dichlorophenol, which comprises passing 2,4-dichlorophenol in the gas phase over a zeolite catalyst.

7. The process as claimed in any one of claims 1 to 6, wherein the zeolite used is of the pentasil, mordenite or faujasite type.

8. The process as claimed in any one of claims 1 to 6, wherein the zeolite used is acid and of the pentasil, mordenite or faujasite type.

9. The process as claimed in any one of claims 1 to 6, wherein the zeolite used is acid and of the pentasil type.

10. The process as claimed in either of claims 8 or 9, wherein the cations in the acid zeolite are protons.

11. The process as claimed in any one of claims 1 to 10, wherein the operating temperature is between 300 and 550°C.


**Revendications**

1. Procédé pour isomériser des monochlorophénols ou des dichlorophénols, caractérisé en ce qu'on fait passer au moins un monochlorophénol ou un dichlorophénol en phase gazeuse, sur un catalyseur de type zéolite.

2. Procédé pour isomériser des monochlorophénols, caractérisé en ce qu'on fait passer au moins un chlorophénol en phase gazeuse sur un catalyseur de type zéolite.

3. Procédé selon la revendication 2, en ce que l'on utilise des mélanges du chloro-2 phénol et du chloro-4 phénol.

4. Procédé pour produire du chloro-3 phénol, caractérisé en ce qu'on fait passer du chloro-2 phénol et/ou du chloro-4 phénol en phase gazeuse sur un catalyseur de type zéolite.

5. Procédé pour isomériser des dichlorophénols, caractérisé en ce qu'on fait passer au moins un dichlorophénol en phase gazeuse sur un catalyseur de type zéolite.

6. Procédé pour produire du dichloro-2,5 phénol, caractérisé en ce qu'on fait passer du dichloro-2,4 phénol en phase gazeuse sur un catalyseur de type zéolite.

7. Procédé selon l'une quelconque des revendications 1 à 6, caractérisé en ce qu'on utilise une zéolite du type pentasil, mordénite ou faujasite.

8. Procédé selon l'une quelconque des revendications 1 à 6, caractérisé en ce qu'on utilise une zéolite acide du type pentasil, mordénite ou faujasite.

9. Procédé selon l'une quelconque des revendications 1 à 6, caractérisé en ce qu'on utilise une zéolite acide du type pentasil.

10. Procédé selon l'une des revendications 8 et 9, caractérisé en que la zéolite acide contient des protons comme cations.

11. Procédé selon l'une quelconque des revendications 1 à 10, caractérisé en ce qu'on travaille à des températures comprises entre 300 et 550°C.